# EUROPEAN PATENT SPECIFICATION

(11) **EP 2 755 680 B1**
(45) Date of publication and mention of the grant of the patent: **04.07.2018**
(21) Application number: 12831495.2
(22) Date of filing: 12.09.2012
(51) Int. Cl.: A61K 39/00, A61K 45/00

(54) **PARTICULATE VACCINE FORMULATIONS**
PARTIKELFÖRMIGE IMPFSTOFFFORMULIERUNGEN
FORMULATIONS VACCINALES PARTICULAIRES

(30) Priority: 12.09.2011 US 201161533512 P
(43) Date of publication of application: 23.07.2014
(73) Proprietor: PDS Biotechnology Corporation, North Brunswick, NJ 08902 (US)
(72) Inventor: BEDU-ADDO, Frank, Bethel Connecticut 06801 (US); CONN, Gregory, 28020 Madrid (ES); JACOBSON, Eric, Cincinnati Ohio 45236 (US); MERCER, Carol, Mason Ohio 45040 (US); JOHNSON, Kenya, Mason Ohio 45040 (US)
(74) Representative: Ponti & Partners, S.L.P
(86) International application number: PCT/US2012/054786
(87) International publication number: WO 2013/039989

(56) References cited:
- WO-A1-95/27508
- WO-A1-2009/129227
- WO-A1-2009/129227
- WO-A2-2007/022152
- WO-A2-2008/116078
- US-A1- 2008 131 455
- US-A1- 2008 131 455
- US-A1- 2008 248 044
- ELIZABETH A. VASIEVICH ET AL: "Enantiospecific adjuvant activity of cationic lipid DOTAP in cancer vaccine", CANCER IMMUNOLOGY, IMMUNOTHERAPY, vol. 60, no. 5, 1 May 2011 (2011-05-01), pages 629-638, XP055057926, ISSN: 0340-7004, DOI: 10.1007/s00262-011-0970-1
- WEIHSU CHEN ET AL: "A simple but effective cancer vaccine consisting of an antigen and a cationic lipid", CANCER IMMUNOLOGY, IMMUNOTHERAPY, SPRINGER, BERLIN, DE, vol. 57, no. 4, 28 August 2007 (2007-08-28), pages 517-530, XP019586704, ISSN: 1432-0851
- BEI R ET AL: "The use of a cationic liposome formulation (DOTAP) mixed with a recombinant tumor-associated antigen to induce immune responses and protective immunity in mice", JOURNAL OF IMMUNOTHERAPY, LIPPINCOTT WILLIAMS & WILKINS, HAGERSTOWN, MD, US, vol. 21, no. 3, 1 January 1998 (1998-01-01), pages 159-169, XP002963675, ISSN: 1524-9557
- VASIEVICH ET AL.: 'Enantiospecific adjuvant activity of cationic lipid DOTAP in cancer vaccine.' CANCER IMMUNOL IMMUNOTHER vol. 60, no. 5, May 2011, pages 629 - 638, XP055057926
- I.W. HAMLEY: "Self-assembly of amphiphilic peptides", SOFT MATTER, vol. 7, 2011, pages 4122-4138,
- E. KOKKOLI ET AL.: "Self-assembly and applications of biomimetic and bioactive peptide-amphiphiles", SOFT MATTER, vol. 2, 2006, pages 1015-1024,
- ZHANG L ET AL: "Converting Peptides into Drug Leads by Lipidation", CURRENT MEDICINAL CHEMISTRY, vol. 19, no. 11, April 2012 (2012-04), pages 1602-1618, ISSN: 0929-8673(print)

### SEQUENCE LISTING

<110> PDS BIOTECHNOLOGY CORPORATION BEDU-ADDO, Frank CONN, Gregory JACOBSON, Eric MERCER, Carol JOHNSON, Kenya
<120> PARTICULATE VACCINE FORMULATIONS
<130> 50165-222751
<150> 61/533,512
   <151> 2011-09-12
<160> 11
<170> PatentIn version 3.5
<210> 1
   <211> 9
   <212> PRT
   <213> Homo sapiens
<400> 1
   Arg Ala His Tyr Asn Ile Val Thr Phe
   1 5
<210> 2
   <211> 15
   <212> PRT
   <213> Homo sapiens
<400> 2
   Gly Gln Ala Glu Pro Asp Arg Ala His Tyr Asn Ile Val Thr Phe
   1 5 10 15
<210> 3
   <211> 18
   <212> PRT
   <213> Artificial Sequence
<220>
   <223> Elongated peptide
<400> 3
   Lys Ser Ser Gly Gln Ala Glu Pro Asp Arg Ala His Tyr Asn Ile Val
   1 5 10 15
   Thr Phe
<210> 4
   <211> 10
   <212> PRT
   <213> Homo sapiens
<400> 4
   Tyr Met Leu Asp Leu Gln Pro Glu Thr Thr
   1 5 10
<210> 5
   <211> 13
   <212> PRT
   <213> Artificial Sequence
<220>
   <223> Elongated peptide
<400> 5
   Lys Ser Ser Tyr Met Leu Asp Leu Gln Pro Glu Thr Thr
   1 5 10
<210> 6
   <211> 23
   <212> PRT
   <213> Artificial Sequence
<220>
   <223> Elongated peptide
<400> 6
   Lys Ser Ser Met His Gly Asp Thr Pro Thr Leu His Glu Tyr Met Leu
   1               5                   10                  15
   Asp Leu Gln Pro Glu Thr Thr
               20
<210> 7
   <211> 20
   <212> PRT
   <213> Artificial Sequence
<220>
   <223> Elongated peptide
<400> 7
   Lys Ser Ser Leu Leu Met Gly Thr Leu Gly Ile Val Cys Pro Ile Cys
   1               5                   10                  15
   Ser Gln Lys Pro
               20
<210> 8
   <211> 9
   <212> PRT
   <213> Artificial Sequence
<220>
   <223> Elongated peptide
<400> 8
   Lys Val Pro Arg Asn Gln Asp Trp Leu
   1               5
<210> 9
   <211> 9
   <212> PRT
   <213> Homo sapiens
<400> 9
   Ser Tyr Val Asp Phe Phe Val Trp Leu
   1               5
<210> 10
   <211> 9
   <212> PRT
   <213> Artificial Sequence
<220>
   <223> Elongated peptide
<400> 10
   Lys Tyr Ile Cys Asn Ser Ser Cys Met
   1               5
<210> 11
   <211> 12
   <212> PRT
   <213> Artificial Sequence
<220>
   <223> Elongated peptide
<400> 11
   Lys Ser Ser Lys Val Pro Arg Asn Gln Asp Trp Leu
   1               5                   10

## Description

### CROSS-REFERENCE TO RELATED APPLICATIONS

This application claims the benefit under 35 USC § 119(e) of U.S. Provisional Application Serial No. 61/533,512, filed on September 12, 2011.

### TECHNICAL FIELD

Development of safe and effective immunotherapies and therapeutic vaccines for human use remains an important medical need for patients worldwide. Typically, a vaccine formulation includes an antigen to stimulate a targeted immune response. However, some developmental vaccines are ineffective because they are weak stimulators of an immune response in a broad mammalian population. For example, the antigen in the vaccine formulation may be poorly immunogenic in the mammal. In addition, some vaccines may not efficiently deliver antigens to the antigen presenting cells ("APCs") of the mammal's immune system.

Furthermore, some antigens in vaccine formulations are known to be poor stimulators of an immune response in mammals. Other antigens may require processing by the mammalian immune system into a specific antigenic epitope in order to be effective. As a result, delivery of larger amounts of these antigens is necessary. However, delivery of such larger amounts may not be effectively and safely accomplished using nanoparticle delivery systems. Administering protein and peptide antigens in an aqueous solution may also not be beneficial because such antigens are typically weakly immunogenic and poorly taken up by APCs. Although development of antigens into virus-like particles (VLPs) has been successful, VLPs are undesirably expensive to produce and require the use of large recombinant proteins, often requiring fusion proteins and specific, complex assembly techniques. Furthermore, VLPs preclude the use of peptide antigens.

### BACKGROUND AND SUMMARY OF THE INVENTION

Vaccines also typically include adjuvants in an attempt to enhance the efficacy of antigens in the vaccine formulation. For example, adjuvants such as water-in-oil emulsions, alum (e.g., aluminum salts), and other chemicals are typically utilized to enhance antigen response in a mammal. In addition to traditional adjuvants, other adjuvants with intrinsic immune effects (e.g., influenza virosomes and Chiron's MF59) may be used. However, these adjuvants are also undesirable because evidence from animal models (according to clinical trial reports on HSV and influenza vaccines) suggests that they merely enhance production of neutralizing antibodies rather than enhancing T-cell responses in animals.

Document US 2008/248044 A1 discloses polyepitopic peptides from HPV 16 E6 and E7. Said peptides can be formulated as lipopeptides linked to a palmitoyl moiety by means of a K residue. The resulting lipopeptides are formulated into micelles by dispersion in acetic acid solution and said micelles are formulated into a vaccine.

Therefore, there exists a need for new vaccine formulations that effectively deliver antigens or promote antigen uptake by the antigen presenting cells in order to stimulate an immune response in a mammal. Moreover, new and effective methods of stimulating cell mediated immune responses in mammals, possibly by including a safe and effective immunologic modifier ("immunomodulator") in a vaccine formulation, are also very desirable. Accordingly, the present disclosure provides vaccine formulations and method of using the formulations that exhibit desirable properties and provide related advantages for improvement in simplicity, antigen uptake, and the induction of an immune response in a mammal.

The present disclosure provides vaccine formulations comprising at least one peptide antigen assembly and at least one adjuvant. The disclosure also provides methods of inducing an immune response in a mammal and methods of treating a disease in a mammal utilizing the vaccine formulations.

The vaccine formulations and methods according to the present disclosure provide several advantages compared to other formulations and methods in the art. First, the vaccine formulations include an adjuvant that is an immunomodulator to enhance, direct, or promote an appropriate immune response in a mammal. Immunomodulators have the potential to effectively boost a mammal's immune response to antigens if they are included in a vaccine formulation. For example, an immunomodulator may advantageously accomplish one or more of the following: (1) improve antigen delivery and/or processing in the APC, (2) induce the production of immunomodulatory cytokines that favor the development of immune responses to the antigen, thus promoting cell mediated immunity, including cytotoxic T-lymphocytes ("CTL"), (3) reduce the number of immunizations or the amount of antigen required for an effective vaccine, (4) increase the biological or immunological half-life of the vaccine antigen, and (5) overcome immune tolerance to antigen by inhibiting immune suppressive factors. In some embodiments, cationic lipid-based adjuvants may be utilized potent immunomodifying adjuvants and can elicit superior T-cell and antibody immune responses in vaccine formulations.

Second, the vaccine formulations, such as particulate vaccine formulations, include a naturally or self-forming antigen assembly, such as a micelle structure or a bilayer structure, which effectively promotes larger amounts of antigen uptake by APCs compared to traditional vaccine formulations. Such an antigen assembly allows for formulation of antigens in a suitable form to be taken up and processed by APCs in a mammal, resulting in a more potent antigen-specific immune response. Furthermore, the spontaneous formation of the protein or peptide antigens into simple organized particulate structures such as micellar or bilayer structures in aqueous media allows for structures that can be effectively taken up and processed by APCs. Consequently, potent vaccines formulations can be administered in a mixture or in combination with adjuvants.

Third, the peptides or proteins utilized in the antigen assemblies may be modified such that the ratio of hydrophilic to lipophilic groups enables the formation of bilayer or micellar structures. Modification of the protein or peptide antigen may be achieved by various means, such as by attaching a lipophilic group (e.g., a hydrocarbon chain or a hydrophobic amino acid sequence) to a hydrophilic peptide and vice versa with a hydrophobic protein or peptide. The size of the attached groups may also be modified based on the size of the peptide and extent of hydrophobicity or hydrophilicity desired.

Finally, as demonstrated in the present disclosure, such vaccine formulations result in significantly improved immunogenicity of vaccines compared to administration of identical amounts of antigen and adjuvant via traditional lioposome or micelle encapsulated vaccine formulations.

The following numbered embodiments are contemplated and are non-limiting:
1. A vaccine formulation comprising an adjuvant and an antigen assembly.
2. The vaccine formulation of clause 1, wherein the formulation is a particulate vaccine formulation.
3. The vaccine formulation of clause 1 or clause 2, wherein the adjuvant and the antigen assembly are a mixture.
4. The vaccine formulation of any one of clauses 1 to 3, wherein the adjuvant is an immunomodulator.
5. The vaccine formulation of any one of clauses 1 to 4, wherein the adjuvant is a nanoparticle.
6. The vaccine formulation of any one of clauses 1 to 5, wherein the adjuvant is a cationic lipid.
7. The vaccine formulation of clause 6, wherein the cationic lipid is purified.
8. The vaccine formulation of clause 6 or clause 7, wherein the cationic lipid is selected from the group consisting of DOTAP, DOTMA, DOEPC, and combinations thereof.
9. The vaccine formulation of any one of clauses 6 to 8, wherein the cationic lipid is DOTAP.
10. The vaccine formulation of any one of clauses 6 to 8, wherein the cationic lipid is DOTMA.
11. The vaccine formulation of any one of clauses 6 to 8, wherein the cationic lipid is DOEPC.
12. The vaccine formulation of any one of clauses 1 to 5, wherein the adjuvant is an enantiomer of a cationic lipid.
13. The vaccine formulation of clause 12, wherein the enantiomer is purified.
14. The vaccine formulation of clause 12 or clause 13, wherein the enantiomer is R-DOTAP or S-DOTAP.
15. The vaccine formulation of any one of clauses 12 to 14, wherein the enantiomer is R-DOTAP.
16. The vaccine formulation of any one of clauses 12 to 14, wherein the enantiomer is S-DOTAP.
17. The vaccine formulation of any one of clauses 1 to 16, wherein the antigen assembly is a self-assembling structure.
18. The vaccine formulation of any one of clauses 1 to 17, wherein the antigen assembly is a micellar structure.
19. The vaccine formulation of any one of clauses 1 to 17, wherein the antigen assembly is a lipid bilayer structure.
20. The vaccine formulation of any one of clauses 1 to 19, wherein the antigen assembly is a tubular structure.
21. The vaccine formulation of any one of clauses 1 to 19, wherein the antigen assembly is a spherical structure.
22. The vaccine formulation of any one of clauses 1 to 21, wherein the antigen assembly comprises one or more antigens.
23. The vaccine formulation of any one of clauses 1 to 22, wherein one or more antigens is a protein-based antigen.
24. The vaccine formulation of any one of clauses 1 to 23, wherein one or more antigens is a peptide-based antigen.
25. The vaccine formulation of any one of clauses 1 to 24, wherein one or more antigens is selected from the group consisting of a cancer antigen, a viral antigen, a bacterial antigen, and a pathogenic antigen.
26. The vaccine formulation of any one of clauses 1 to 25, wherein one or more antigens is a viral antigen.
27. The vaccine formulation of any one of clauses 1 to 26, wherein one or more antigens is a bacterial antigen.
28. The vaccine formulation of any one of clauses 1 to 27, wherein one or more antigens is a pathogenic antigen.
29. The vaccine formulation of clause 28, wherein the pathogenic antigen is a synthetic or recombinant antigen.
30. The vaccine formulation of any one of clauses 1 to 29, wherein at least one antigen is an HPV protein or peptide.
31. The vaccine formulation of any one of clauses 1 to 30, wherein at least one antigen is a melanoma antigen.
32. The vaccine formulation of clause 31, wherein the melanoma antigen is selected from the group comprising of gp100 (KVPRNQDWL [SEQ. ID. No. 8]), TRP2 (SYVDFFVWL [SEQ. ID. No. 9]), and p53 (KYICNSSCM [SEQ. ID. No. 10]), and combinations thereof.
33. The vaccine formulation of any one of clauses 1 to 32, wherein at least one antigen is selected from the group consisting of a lipoprotein, a lipopeptide, and a protein or peptide modified with an amino acid sequence having an increased hydrophobicity or a decreased hydrophobicity.
34. The vaccine formulation of any one of clauses 1 to 33, wherein one or more antigens is a lipidated antigen or an antigen modified to increase hydrophobicity of the antigen.
35. The vaccine formulation of any one of clauses 1 to 34, wherein at least one antigen is a modified protein or peptide.
36. The vaccine formulation of clause 35, wherein the modified protein or peptide is bonded to a hydrophobic group.
37. The vaccine formulation of clause 35 or clause 36, wherein the modified protein or peptide bonded to a hydrophobic group further comprises a linker sequence between the antigen and the hydrophobic group.
38. The vaccine formulation of clause 37, wherein the hydrophobic group is a palmitoyl group.
39. The vaccine formulation of any one of clauses 1 to 38, wherein at least one antigen is an unmodified protein or peptide.
40. The vaccine formulation of any one of clauses 1 to 39, wherein at least one antigen is selected from the group consisting of RAHYNIVTF (SEQ. ID. NO: 1), GQAEPDRAHYNIVTF (SEQ. ID. NO: 2), KSSGQAEPDRAHYNIVTF (SEQ. ID. NO: 3), YMLDLQPETT (SEQ. ID. NO: 4), KSSYMLDLQPETT (SEQ. ID. NO: 5), KSSMHGDTPTLHEYMLDLQPETT (SEQ. ID. NO: 6), KSSLLMGTLGIVCPICSQKP (SEQ. ID. NO: 7), KVPRNQDWL (SEQ. ID. NO: 8), SYVDFFVWL (SEQ. ID. NO: 9), KYICNSSCM (SEQ. ID. NO: 10), and KSSKVPRNQDWL (SEQ. ID. NO: 11).
41. The vaccine formulation of any one of clauses 1 to 40, wherein at least one antigen is RAHYNIVTF (SEQ. ID. NO: 1).
42. The vaccine formulation of any one of clauses 1 to 41, wherein at least one antigen is GQAEPDRAHYNIVTF (SEQ. ID. NO: 2).
43. The vaccine formulation of any one of clauses 1 to 42, wherein at least one antigen is KSSGQAEPDRAHYNIVTF (SEQ. ID. NO: 3).
44. The vaccine formulation of clause 43, wherein KSSGQAEPDRAHYNIVTF (SEQ. ID. NO: 3) is modified to further comprise a hydrophobic group.
45. The vaccine formulation of clause 44, wherein the hydrophobic group is a palmitoyl group.
46. The vaccine formulation of any one of clauses 1 to 45, wherein at least one antigen is YMLDLQPETT (SEQ. ID. NO: 4).
47. The vaccine formulation of any one of clauses 1 to 46, wherein at least one antigen is KSSYMLDLQPETT (SEQ. ID. NO: 5).
48. The vaccine formulation of clause 47, wherein KSSYMLDLQPETT (SEQ. ID. NO: 5) is modified to further comprise a hydrophobic group.
49. The vaccine formulation of clause 48, wherein the hydrophobic group is a palmitoyl group.
50. The vaccine formulation of any one of clauses 1 to 49, wherein at least one antigen is KSSMHGDTPTLHEYMLDLQPETT (SEQ. ID. NO: 6).
51. The vaccine formulation of clause 50, wherein KSSMHGDTPTLHEYMLDLQPETT (SEQ. ID. NO: 6) is modified to further comprise a hydrophobic group.
52. The vaccine formulation of clause 51, wherein the hydrophobic group is a palmitoyl group.
53. The vaccine formulation of any one of clauses 1 to 52, wherein at least one antigen is KSSLLMGTLGIVCPICSQKP (SEQ. ID. NO: 7).
54. The vaccine formulation of clause 53, wherein KSSLLMGTLGIVCPICSQKP (SEQ. ID. NO: 7) is modified to further comprise a hydrophobic group.
55. The vaccine formulation of clause 54, wherein the hydrophobic group is a palmitoyl group.
56. The vaccine formulation of any one of clauses 1 to 55, wherein at least one antigen is KVPRNQDWL (SEQ. ID. NO: 8).
57. The vaccine formulation of any one of clauses 1 to 56, wherein at least one antigen is SYVDFFVWL (SEQ. ID. NO: 9).
58. The vaccine formulation of any one of clauses 1 to 57, wherein at least one antigen is KYICNSSCM (SEQ. ID. NO: 10).
59. The vaccine formulation of any one of clauses 1 to 58, wherein at least one antigen is KSSKVPRNQDWL (SEQ. ID. NO: 11).
60. The vaccine formulation of clause 59, wherein KSSKVPRNQDWL (SEQ. ID. NO: 11) is modified to further comprise a hydrophobic group.
61. The vaccine formulation of clause 60, wherein the hydrophobic group is a palmitoyl group.
62. The vaccine formulation of any one of clauses 1 to 61, wherein the formulation induces an immune response in an mammal by activating the mitogen-activated protein (MAP) kinase signaling pathway.
63. The vaccine formulation of clause 62, wherein the MAP kinase signaling pathway is activated by stimulating at least one of extracellular signal-regulated kinase ("ERK")-1, ERK-2, and p38.
64. The vaccine formulation of any one of clauses 1 to 63, wherein the formulation enhances functional antigen-specific CD8+ T lymphocyte response in a mammal.
65. The vaccine formulation of any one of clauses 62 to 64, wherein the mammal is a human.
66. A method of inducing an immune response in a mammal, said method comprising the step of administering an effective amount of a vaccine formulation to the mammal, wherein the vaccine formulation comprises an adjuvant and an antigen assembly.
67. The method of clause 66, wherein the immune response is activated via the MAP kinase signaling pathway in cells of the immune system of the mammal.
68. The method of clause 67, wherein the MAP kinase signaling pathway is activated by stimulating at least one of ERK-1, ERK-2, and p38.
69. The method of any one of clauses 66 to 68, wherein the immune response activates cytotoxic T lymphocytes in the mammal.
70. The method of clause 69, wherein the cytotoxic T lymphocytes are CD8+ T cells.
71. The method of any one of clauses 66 to 70, wherein the administration enhances functional antigen-specific CD8+ T lymphocyte response in the mammal.
72. The method of any one of clauses 66 to 71, wherein the immune response activates an antibody response in the mammal.
73. The method of any one of clauses 66 to 72, wherein the immune response activates interferon-gamma (IFN-γ) in the mammal.
74. The method of any one of clauses 66 to 73, wherein the formulation is a particulate vaccine formulation.
75. The method of any one of clauses 66 to 74, wherein the adjuvant and the antigen assembly are a mixture.
76. The method of any one of clauses 66 to 75, wherein the adjuvant is an immunomodulator.
77. The method of any one of clauses 66 to 76, wherein the adjuvant is a nanoparticle.
78. The method of any one of clauses 66 to 77, wherein the adjuvant is a cationic lipid.
79. The method of clause 78, wherein the cationic lipid is purified.
80. The method of clause 78 or clause 79, wherein the cationic lipid is selected from the group consisting of DOTAP, DOTMA, DOEPC, and combinations thereof.
81. The method of any one of clauses 78 to 80, wherein the cationic lipid is DOTAP.
82. The method of any one of clauses 78 to 80, wherein the cationic lipid is DOTMA.
83. The method of any one of clauses 78 to 80, wherein the cationic lipid is DOEPC.
84. The method of any one of clauses 66 to 78, wherein the adjuvant is an enantiomer of a cationic lipid.
85. The method of clause 84, wherein the enantiomer is purified.
86. The method of clause 84 or clause 85, wherein the enantiomer is R-DOTAP or S-DOTAP.
87. The method of any one of clauses 84 to 86, wherein the enantiomer is R-DOTAP.
88. The method of any one of clauses 84 to 86, wherein the enantiomer is S-DOTAP.
89. The method of any one of clauses 66 to 88, wherein the antigen assembly is a self-assembling structure.
90. The method of any one of clauses 66 to 89, wherein the antigen assembly is a micellar structure.
91. The method of any one of clauses 66 to 89, wherein the antigen assembly is a lipid bilayer structure.
92. The method of any one of clauses 66 to 91, wherein the antigen assembly is a tubular structure.
93. The method of any one of clauses 66 to 91, wherein the antigen assembly is a spherical structure.
94. The method of any one of clauses 66 to 93, wherein the antigen assembly comprises one or more antigens.
95. The method of clause 94, wherein one or more antigens is a protein-based antigen.
96. The method of clause 94, wherein one or more antigens is a peptide-based antigen.
97. The method of any one of clauses 94 to 96, wherein one or more antigens is selected from the group consisting of a cancer antigen, a viral antigen, a bacterial antigen, and a pathogenic antigen.
98. The method of any one of clauses 94 to 97, wherein one or more antigens is a viral antigen.
99. The method of any one of clauses 94 to 97, wherein one or more antigens is a bacterial antigen.
100. The method of any one of clauses 94 to 97, wherein one or more antigens is a pathogenic antigen.
101. The method of clause 100, wherein the pathogenic antigen is a synthetic or recombinant antigen.
102. The method of any one of clauses 94 to 101, wherein at least one antigen is an HPV protein or peptide.
103. The method of any one of clauses 94 to 102, wherein at least one antigen is a melanoma antigen.
104. The method of clause 103, wherein the melanoma antigen is selected from the group comprising of gp100 (KVPRNQDWL [SEQ. ID. No. 8]), TRP2 (SYVDFFVWL [SEQ. ID. No. 9]), and p53 (KYICNSSCM [SEQ. ID. No. 10]), and combinations thereof.
105. The method of any one of clauses 94 to 104, wherein at least one antigen is selected from the group consisting of a lipoprotein, a lipopeptide, and a protein or peptide modified with an amino acid sequence having an increased hydrophobicity or a decreased hydrophobicity.
106. The method of any one of clauses 94 to 105, wherein one or more antigens is a lipidated antigen or an antigen modified to increase hydrophobicity of the antigen.
107. The method of any one of clauses 94 to 106, wherein at least one antigen is a modified protein or peptide.
108. The method of clause 107, wherein the modified protein or peptide is bonded to a hydrophobic group.
109. The method of clause 107 or clause 108, wherein the modified protein or peptide bonded to a hydrophobic group further comprises a linker sequence between the antigen and the hydrophobic group.
110. The method of clause 109, wherein the hydrophobic group is a palmitoyl group.
111. The method of any one of clauses 94 to 110, wherein at least one antigen is an unmodified protein or peptide.
112. The method of any one of clauses 94 to 111, wherein at least one antigen is selected from the group consisting of RAHYNIVTF (SEQ. ID. NO: 1), GQAEPDRAHYNIVTF (SEQ. ID. NO: 2), KSSGQAEPDRAHYNIVTF (SEQ. ID. NO: 3), YMLDLQPETT (SEQ. ID. NO: 4), KSSYMLDLQPETT (SEQ. ID. NO: 5), KSSMHGDTPTLHEYMLDLQPETT (SEQ. ID. NO: 6), KSSLLMGTLGIVCPICSQKP (SEQ. ID. NO: 7), KVPRNQDWL (SEQ. ID. NO: 8), SYVDFFVWL (SEQ. ID. NO: 9), KYICNSSCM (SEQ. ID. NO: 10), and KSSKVPRNQDWL (SEQ. ID. NO: 11).
113. The method of any one of clauses 94 to 112, wherein at least one antigen is RAHYNIVTF (SEQ. ID. NO: 1).
114. The method of any one of clauses 94 to 113, wherein at least one antigen is GQAEPDRAHYNIVTF (SEQ. ID. NO: 2).
115. The method of any one of clauses 94 to 114, wherein at least one antigen is KSSGQAEPDRAHYNIVTF (SEQ. ID. NO: 3).
116. The method of clause 115, wherein KSSGQAEPDRAHYNIVTF (SEQ. ID. NO: 3) is modified to further comprise a hydrophobic group.
117. The method of clause 116, wherein the hydrophobic group is a palmitoyl group.
118. The method of any one of clauses 94 to 117, wherein at least one antigen is YMLDLQPETT (SEQ. ID. NO: 4).
119. The method of any one of clauses 94 to 118, wherein at least one antigen is KSSYMLDLQPETT (SEQ. ID. NO: 5).
120. The method of clause 119, wherein KSSYMLDLQPETT (SEQ. ID. NO: 5) is modified to further comprise a hydrophobic group.
121. The method of clause 120, wherein the hydrophobic group is a palmitoyl group.
122. The method of any one of clauses 94 to 121, wherein at least one antigen is KSSMHGDTPTLHEYMLDLQPETT (SEQ. ID. NO: 6).
123. The method of clause 122, wherein KSSMHGDTPTLHEYMLDLQPETT (SEQ. ID. NO: 6) is modified to further comprise a hydrophobic group.
124. The method of clause 123, wherein the hydrophobic group is a palmitoyl group.
125. The method of any one of clauses 94 to 124, wherein at least one antigen is KSSLLMGTLGIVCPICSQKP (SEQ. ID. NO: 7).
126. The method of clause 125, wherein KSSLLMGTLGIVCPICSQKP (SEQ. ID. NO: 7) is modified to further comprise a hydrophobic group.
127. The method of clause 126, wherein the hydrophobic group is a palmitoyl group.
128. The method of any one of clauses 94 to 127, wherein at least one antigen is KVPRNQDWL (SEQ. ID. NO: 8).
129. The method of any one of clauses 94 to 128, wherein at least one antigen is SYVDFFVWL (SEQ. ID. NO: 9).
130. The method of any one of clauses 94 to 129, wherein at least one antigen is KYICNSSCM (SEQ. ID. NO: 10).
131. The method of any one of clauses 94 to 130, wherein at least one antigen is KSSKVPRNQDWL (SEQ. ID. NO: 11).
132. The method of clause 131, wherein KSSKVPRNQDWL (SEQ. ID. NO: 11) is modified to further comprise a hydrophobic group.
133. The method of clause 132, wherein the hydrophobic group is a palmitoyl group.
134. The method of any one of clauses 66 to 133, wherein the mammal is a human.
135. A method of treating a disease in a mammal, said method comprising the step of administering an effective amount of a vaccine formulation to the mammal, wherein the vaccine formulation comprises an adjuvant and an antigen assembly.
136. The method of clause 135, wherein the method is a prophylactic treatment.
137. The method of clause 135, wherein the disease is a cancer.
138. The method of any one of clauses 135 to 137, wherein the administration activates an immune response via the MAP kinase signaling pathway in cells of the immune system of the mammal.
139. The method of clause 138, wherein the MAP kinase signaling pathway is activated by stimulating at least one of ERK-1, ERK-2, and p38.
140. The method of any one of clauses 135 to 139, wherein the immune response activates cytotoxic T lymphocytes in the mammal.
141. The method of clause 140, wherein the cytotoxic T lymphocytes are CD8+ T cells.
142. The method of any one of clauses 135 to 141, wherein the immune response activates an antibody response in the mammal.
143. The method of any one of clauses 135 to 142, wherein the immune response activates interferon-gamma (IFN-γ) in the mammal.
144. The method of any one of clauses 135 to 143, wherein the administration enhances functional antigen-specific CD8+ T lymphocyte response.
145. The method of any one of clauses 135 to 144, wherein the formulation is a particulate vaccine formulation.
146. The method of any one of clauses 135 to 145, wherein the adjuvant and the antigen assembly are a mixture.
147. The method of any one of clauses 135 to 146, wherein the adjuvant is an immunomodulator.
148. The method of any one of clauses 135 to 147, wherein the adjuvant is a nanoparticle.
149. The method of any one of clauses 135 to 148, wherein the adjuvant is a cationic lipid.
150. The method of clause 149, wherein the cationic lipid is purified.
151. The method of clause 149 or clause 150, wherein the cationic lipid is selected from the group consisting of DOTAP, DOTMA, DOEPC, and combinations thereof.
152. The method of any one of clauses 149 to 151, wherein the cationic lipid is DOTAP.
153. The method of any one of clauses 149 to 151, wherein the cationic lipid is DOTMA.
154. The method of any one of clauses 149 to 151, wherein the cationic lipid is DOEPC.
155. The method of any one of clauses 135 to 148, wherein the adjuvant is an enantiomer of a cationic lipid.
156. The method of clause 155, wherein the enantiomer is purified.
157. The method of clause 155 or clause 156, wherein the enantiomer is R-DOTAP or S-DOTAP.
158. The method of any one of clauses 155 to 157, wherein the enantiomer is R-DOTAP.
159. The method of any one of clauses 155 to 157, wherein the enantiomer is S-DOTAP.
160. The method of any one of clauses 135 to 159, wherein the antigen assembly is a self-assembling structure.
161. The method of any one of clauses 135 to 160, wherein the antigen assembly is a micellar structure.
162. The method of any one of clauses 135 to 160, wherein the antigen assembly is a lipid bilayer structure.
163. The method of any one of clauses 135 to 162, wherein the antigen assembly is a tubular structure.
164. The method of any one of clauses 135 to 162, wherein the antigen assembly is a spherical structure.
165. The method of any one of clauses 135 to 164, wherein the antigen assembly comprises one or more antigens.
166. The method of clause 165, wherein one or more antigens is a protein-based antigen.
167. The method of clause 165 or clause 166, wherein one or more antigens is a peptide-based antigen.
168. The method of any one of clauses 165 to 167, wherein one or more antigens is selected from the group consisting of a cancer antigen, a viral antigen, a bacterial antigen, and a pathogenic antigen.
169. The method of any one of clauses 165 to 168, wherein one or more antigens is a viral antigen.
170. The method of any one of clauses 165 to 168, wherein one or more antigens is a bacterial antigen.
171. The method of any one of clauses 165 to 168, wherein one or more antigens is a pathogenic antigen.
172. The method of any one of clauses 165 to 168, wherein the pathogenic antigen is a synthetic or recombinant antigen.
173. The method of any one of clauses 165 to 172, wherein at least one antigen is an HPV protein or peptide.
174. The method of any one of clauses 165 to 173, wherein at least one antigen is a melanoma antigen.
175. The method of clause 174, wherein the melanoma antigen is selected from the group comprising of gp100 (KVPRNQDWL [SEQ. ID. No. 8]), TRP2 (SYVDFFVWL [SEQ. ID. No. 9]), and p53 (KYICNSSCM [SEQ. ID. No. 10]), and combinations thereof.
176. The method of any one of clauses 165 to 175, wherein at least one antigen is selected from the group consisting of a lipoprotein, a lipopeptide, and a protein or peptide modified with an amino acid sequence having an increased hydrophobicity or a decreased hydrophobicity.
177. The method of any one of clauses 165 to 176, wherein one or more antigens is a lipidated antigen or an antigen modified to increase hydrophobicity of the antigen.
178. The method of any one of clauses 165 to 177, wherein at least one antigen is a modified protein or peptide.
179. The method of clause 178, wherein the modified protein or peptide is bonded to a hydrophobic group.
180. The method of clause 178, wherein the modified protein or peptide bonded to a hydrophobic group further comprises a linker sequence between the antigen and the hydrophobic group.
181. The method of clause 180, wherein the hydrophobic group is a palmitoyl group.
182. The method of any one of clauses 165 to 181, wherein at least one antigen is an unmodified protein or peptide.
183. The method of any one of clauses 165 to 182, wherein at least one antigen is selected from the group consisting of RAHYNIVTF (SEQ. ID. NO: 1), GQAEPDRAHYNIVTF (SEQ. ID. NO: 2), KSSGQAEPDRAHYNIVTF (SEQ. ID. NO: 3), YMLDLQPETT (SEQ. ID. NO: 4), KSSYMLDLQPETT (SEQ. ID. NO: 5), KSSMHGDTPTLHEYMLDLQPETT (SEQ. ID. NO: 6), KSSLLMGTLGIVCPICSQKP (SEQ. ID. NO: 7), KVPRNQDWL (SEQ. ID. NO: 8), SYVDFFVWL (SEQ. ID. NO: 9), KYICNSSCM (SEQ. ID. NO: 10), and KSSKVPRNQDWL (SEQ. ID. NO: 11).
184. The method of any one of clauses 165 to 183, wherein at least one antigen is RAHYNIVTF (SEQ. ID. NO: 1).
185. The method of any one of clauses 165 to 184, wherein at least one antigen is GQAEPDRAHYNIVTF (SEQ. ID. NO: 2).
186. The method of any one of clauses 165 to 185, wherein at least one antigen is KSSGQAEPDRAHYNIVTF (SEQ. ID. NO: 3).
187. The method of clause 186, wherein KSSGQAEPDRAHYNIVTF (SEQ. ID. NO: 3) is modified to further comprise a hydrophobic group.
188. The method of clause 187, wherein the hydrophobic group is a palmitoyl group.
189. The method of any one of clauses 165 to 188, wherein at least one antigen is YMLDLQPETT (SEQ. ID. NO: 4).
190. The method of any one of clauses 165 to 189, wherein at least one antigen is KSSYMLDLQPETT (SEQ. ID. NO: 5).
191. The method of clause 190, wherein KSSYMLDLQPETT (SEQ. ID. NO: 5) is modified to further comprise a hydrophobic group.
192. The method of clause 191, wherein the hydrophobic group is a palmitoyl group.
193. The method of any one of clauses 165 to 192, wherein at least one antigen is KSSMHGDTPTLHEYMLDLQPETT (SEQ. ID. NO: 6).
194. The method of clause 193, wherein KSSMHGDTPTLHEYMLDLQPETT (SEQ. ID. NO: 6) is modified to further comprise a hydrophobic group.
195. The method of clause 194, wherein the hydrophobic group is a palmitoyl group.
196. The method of any one of clauses 165 to 195, wherein at least one antigen is KSSLLMGTLGIVCPICSQKP (SEQ. ID. NO: 7).
197. The method of clause 196, wherein KSSLLMGTLGIVCPICSQKP (SEQ. ID. NO: 7) is modified to further comprise a hydrophobic group.
198. The method of clause 197, wherein the hydrophobic group is a palmitoyl group.
199. The method of any one of clauses 165 to 198, wherein at least one antigen is KVPRNQDWL (SEQ. ID. NO: 8).
200. The method of any one of clauses 165 to 199, wherein at least one antigen is SYVDFFVWL (SEQ. ID. NO: 9).
201. The method of any one of clauses 165 to 200, wherein at least one antigen is KYICNSSCM (SEQ. ID. NO: 10).
202. The method of any one of clauses 165 to 201, wherein at least one antigen is KSSKVPRNQDWL (SEQ. ID. NO: 11).
203. The method of clause 202, wherein KSSKVPRNQDWL (SEQ. ID. NO: 11) is modified to further comprise a hydrophobic group.
204. The method of clause 203, wherein the hydrophobic group is a palmitoyl group.
205. The method of any one of clauses 135 to 204, wherein the mammal is a human.

### BRIEF DESCRIPTION OF THE DRAWINGS

FIGURE 1 shows the anti-tumor immune response of various cationic lipid adjuvants coupled with the HPV-16 E7 peptide antigen compared to traditional adjuvants similarly formulated with the E7 antigen.
FIGURE 2 shows tumor regression efficacy with various liposomally encapsulated formulations of R-DOTAP/pE7₄₃₋₅₇ complex compared with the R-DOTAP/pE7₄₉₋₅₇ complex where amino acids 43 to 48 are absent from the antigenic region.
FIGURE 3 shows tumor regression efficacy using a mixture of modified HVP-16 E7₄₃₋₅₇ Micelles with R-DOTAP or S-DOTAP liposomal adjuvant nanoparticles compared to empty R-DOTAP liposome nanoparticles.
FIGURE 4 shows a negative stain electron microscopy image of a vaccine formulation containing cylindrical pE7_{43-57M} micelles composed of palmitoyl-KSSGQAEPDRAHYNIVTF [SEQ. ID. No. 3] and spherical cationic lipid R-DOTAP nanoparticles.
FIGURE 5 shows a negative stain electron microscopy image of a vaccine formulation containing a mixture of an antigen assembly in spherical micelle structures comprising palmitoyl-KSSYMLDLQPETT [SEQ. ID. NO: 5) and an adjuvant comprising spherical R-DOTAP liposome nanoparticles co-existing in the formulated mixture.
FIGURE 6 shows a negative stain electron microscopy image of a vaccine formulation containing cylindrical structures composed of pE7_{1-20M} or palmitoyl - KSSMHGDTPTLHEYMLDLQPETT [SEQ. ID. No. 6] and spherical cationic lipid R-DOTAP nanoparticles.
FIGURE 7 shows results of an ELISPOT study comparing the antigen-specific immune response to the melanoma peptide, gp100, in vaccine formulations containing various melanoma antigens encapsulated in a R-DOTAP adjuvant and a melanoma peptide micellar formulation co-administered with the R-DOTAP liposome adjuvant.
FIGURE 8 shows results of an ELISPOT study comparing the antigen-specific immune response to the HPV-16 peptide formulated at identical doses as a micelle and co-administered with various adjuvants versus the HPV-16 peptide encapsulated in the liposome adjuvants.

The present invention relates to a vaccine formulation according to the set of claims.

Various embodiments of the invention are described herein as follows. In one embodiment described herein, a vaccine formulation is provided. The vaccine formulation comprises an adjuvant and an antigen assembly.

In another embodiment, a method of inducing an immune response in a mammal is provided. The method comprises the step of administering an effective amount of a vaccine formulation to the mammal, wherein the vaccine formulation comprises an adjuvant and an antigen assembly

In yet another embodiment, a method of treating a disease in a mammal is provided. The method comprises the step of administering an effective amount of a vaccine formulation to the mammal, wherein the vaccine formulation comprises an adjuvant and an antigen assembly.

In the various embodiments, the vaccine formulation comprises an adjuvant and an antigen assembly. As used herein, the term "adjuvant" refers to a substance that enhances, augments and/or potentiates a mammal's immune response to an antigen. As used herein, the term "antigen assembly" refers to a composition containing one or more antigens.

In some embodiments described herein, the vaccine formulation is a particulate vaccine formulation. In some embodiments, the adjuvant and the antigen assembly are a mixture.

In some embodiments described herein, the adjuvant is an immunomodulator. As used herein, the term "immunomodulator" refers to an immunologic modifier that enhances, directs, and/or promotes an immune response in a mammal.

In some embodiments described herein, the adjuvant is a nanoparticle. As used herein, the term "nanoparticle" refers to a particle having a size measured on the nanometer scale. As used herein, the "nanoparticle" refers to a particle having a structure with a size of less than about 1,000 nanometers. In some embodiments, the nanoparticle is a liposome.

In some embodiments described herein, the adjuvant is a cationic lipid. As used herein, the term "cationic lipid" refers to any of a number of lipid species which carry a net positive charge at physiological pH or have a protonatable group and are positively charged at pH lower than the pKa.

Suitable cationic lipid according to the present disclosure include, but are not limited to: 3-.beta.[.sup.4N-(.sup.lN, .sup.8-diguanidino spermidine)-carbamoyl]cholesterol (BGSC); 3-.beta. [N,N-diguanidinoethyl-aminoethane)-carbamoyl]cholesterol (BGTC); N,N.sup. 1N.sup.2N.sup.3Tetra-methyltetrapalmitylspermine (cellfectin); N-t-butyl-N'-tetradecyl-3-tetradecyl-aminopropion-amidine (CLONfectin); dimethyldioctadecyl ammonium bromide (DDAB); 1,2-dimyristyloxypropyl-3-dimethyl-hydroxy ethyl ammonium bromide (DMRIE); 2,3-dioleoyloxy-N-[2(sperminecarboxamido)ethyl]-N,N-dimethyl-1-p- - ropanaminium trifluorocetate) (DOSPA); 1,3-dioleoyloxy-2-(6-carboxyspermyl)-propyl amide (DOSPER); 4-(2,3-bis-palmitoyloxy-propyl)-1-methyl-1H-imidazole (DPIM) N,N,N',N'-tetramethyl-N,N'-bis(2-hydroxyethyl)-2,3-dioleoyloxy-1,4-butane- diammonium iodide) (Tfx-50); N-1-(2,3-dioleoyloxy) propyl-N,N,N-trimethyl ammonium chloride (DOTMA) or other N-(N,N-1-dialkoxy)-alkyl-N,N,N-trisubstituted ammonium surfactants; 1,2 dioleoyl-3-(4'-trimethylammonio) butanol-sn-glycerol (DOBT) or cholesteryl (4'trimethylammonia) butanoate (ChOTB) where the trimethylammonium group is connected via a butanol spacer arm to either the double chain (for DOTB) or cholesteryl group (for ChOTB); DORI (DL-1,2-dioleoyl-3-dimethylaminopropyl-.beta.-hydroxyethylammonium) or DORIE (DL-1,2-O-dioleoyl-3-dimethylaminopropyl-.beta.-hydroxyethylammoniu- -m) (DORIE) or analogs thereof as disclosed in WO 93/03709; 1,2-dioleoyl-3-succinyl-sn-glycerol choline ester (DOSC); cholesteryl hemisuccinate ester (ChOSC); lipopolyamines such as dioctadecylamidoglycylspermine (DOGS) and dipalmitoyl phosphatidylethanolamylspermine (DPPES), cholesteryl-3.beta.-carboxyl-amido-ethylenetrimethylammonium iodide, 1-dimethylamino-3-trimethylammonio-DL-2-propyl-cholesteryl carboxylate iodide, cholesteryl-3-O-carboxyamidoethyleneamine, cholesteryl-3-.beta.-oxysuccinamido-ethylenetrimethylammonium iodide, 1-dimethylamino-3-trimethylammonio-DL-2-propyl-cholesteryl-3-.beta.-oxysu- ccinate iodide, 2-(2-trimethylammonio)-ethylmethylamino ethyl-cholesteryl-3-.beta.-oxysuccinate iodide, 3-.beta.-N-(N',N'-dimethylaminoethane) carbamoyl cholesterol (DC-chol), and 3-.beta.-N-(polyethyleneimine)-carbamoylcholesterol; O,O'-dimyristyl-N-lysyl aspartate (DMKE); O,O'-dimyristyl-N-lysyl-glutamate (DMKD); 1,2-dimyristyloxypropyl-3-dimethyl-hydroxy ethyl ammonium bromide (DMRIE); 1,2-dilauroyl-sn-glycero-3-ethylphosphocholine (DLEPC); 1,2-dimyristoyl-sn-glycero-3-ethylphosphocholine (DMEPC); 1,2-dioleoyl-sn-glycero-3-ethylphosphocholine (DOEPC); 1,2-dipalmitoyl-sn-glycero-3-ethylphosphocholine (DPEPC); 1,2-distearoyl-sn-glycero-3-ethylphosphocholine (DSEPC); 1,2-dioleoyl-3-trimethylammonium propane (DOTAP); dioleoyl dimethylaminopropane (DODAP); 1,2-palmitoyl-3-trimethylammonium propane (DPTAP); 1,2-distearoyl-3-trimethylammonium propane (DSTAP), 1,2-myristoyl-3-trimethylammonium propane (DMTAP); and sodium dodecyl sulfate (SDS). Furthermore, structural variants and derivatives of the any of the described cationic lipids are also contemplated.

In some embodiment, the cationic lipid is selected from the group consisting of DOTAP, DOTMA, DOEPC, and combinations thereof. In other embodiments, the cationic lipid is DOTAP. In yet other embodiments, the cationic lipid is DOTMA. In other embodiments, the cationic lipid is DOEPC. In some embodiments, the cationic lipid is purified.

In some embodiments, the cationic lipid is an enantiomer of a cationic lipid. The term "enantiomer" refers to a stereoisomer of a cationic lipid which is a non-superimposable mirror image of its counterpart stereoisomer, for example R and S enantiomers. In various examples, the enantiomer is R-DOTAP or S-DOTAP. In one example, the enantiomer is R-DOTAP. In another example, the enantiomer is S-DOTAP. In some embodiments, the enantiomer is purified.

In various embodiments described herein, the antigen assembly is a self-assembling structure. In various embodiments described herein, the antigen assembly is a micellar structure. As used herein, the term "micellar" refers to an aggregation of molecules, such as in a colloidal system. In other embodiments, the antigen assembly is a lipid bilayer structure. In some embodiments, the antigen assembly is a tubular structure. In yet other embodiments, the antigen assembly is a spherical structure.

In various embodiments described herein, the antigen assembly comprises one or more antigens. As used herein, the term "antigen" refers to any agent (e.g., protein, peptide, polysaccharide, glycoprotein, glycolipid, nucleic acid, or combination thereof) that, when introduced into a mammal having an immune system (directly or upon expression as in, e.g., DNA vaccines), is recognized by the immune system of the mammal and is capable of eliciting an immune response. As defined herein, the antigen-induced immune response can be humoral or cell-mediated, or both. An agent is termed "antigenic" when it is capable of specifically interacting with an antigen recognition molecule of the immune system, such as an immunoglobulin (antibody) or T cell antigen receptor (TCR).

In some embodiments, one or more antigens is a protein-based antigen. In other embodiments, one or more antigens is a peptide-based antigen. In various embodiments, one or more antigens is selected from the group consisting of a cancer antigen, a viral antigen, a bacterial antigen, and a pathogenic antigen. A "microbial antigen," as used herein, is an antigen of a microorganism and includes, but is not limited to, infectious virus, infectious bacteria, infectious parasites and infectious fungi. Microbial antigens may be intact microorganisms, and natural isolates, fragments, or derivatives thereof, synthetic compounds which are identical to or similar to naturally-occurring microbial antigens and, preferably, induce an immune response specific for the corresponding microorganism (from which the naturally-occurring microbial antigen originated). In one embodiment, the antigen is a viral antigen. In another embodiment, the antigen is a bacterial antigen. In various embodiments, the antigen is a pathogenic antigen. In some embodiments, the pathogenic antigen is a synthetic or recombinant antigen.

In some embodiments, the antigen is a cancer antigen. A "cancer antigen," as used herein, is a molecule or compound (e.g., a protein, peptide, polypeptide, lipoprotein, lipopeptide, glycoprotein, glycopeptides, lipid, glycolipid, carbohydrate, RNA, and/or DNA) associated with a tumor or cancer cell and which is capable of provoking an immune response (humoral and/or cellular) when expressed on the surface of an antigen presenting cell in the context of an MHC molecule. For example, a cancer antigen may be a tumor-associated antigen. Tumor-associated antigens include self antigens, as well as other antigens that may not be specifically associated with a cancer, but nonetheless enhance an immune response to and/or reduce the growth of a tumor or cancer cell when administered to a mammal. In one embodiment, at least one antigen is an HPV protein or peptide.

In some embodiments, at least one antigen is a melanoma antigen. In one embodiment, the melanoma antigen is selected from the group comprising of gp100 (KVPRNQDWL [SEQ. ID. No. 8]), TRP2 (SYVDFFVWL [SEQ. ID. No. 9]), and p53 (KYICNSSCM [SEQ. ID. No. 10]), and combinations thereof.

In various embodiments, at least one antigen is selected from the group consisting of a lipoprotein, a lipopeptide, and a protein or peptide modified with an amino acid sequence having an increased hydrophobicity or a decreased hydrophobicity. In some embodiments, one or more antigens is an antigen modified to increase hydrophobicity of the antigen. In one embodiment, at least one antigen is a modified protein or peptide. In some embodiments, the modified protein or peptide is bonded to a hydrophobic group. In other embodiments, the modified protein or peptide bonded to a hydrophobic group further comprises a linker sequence between the antigen and the hydrophobic group. In some embodiments, the hydrophobic group is a palmitoyl group. In yet other embodiments, at least one antigen is an unmodified protein or peptide.

In some embodiments of the present disclosure, at least one antigen is selected from the group consisting of RAHYNIVTF (SEQ. ID. NO: 1), GQAEPDRAHYNIVTF (SEQ. ID. NO: 2), KSSGQAEPDRAHYNIVTF (SEQ. ID. NO: 3), YMLDLQPETT (SEQ. ID. NO: 4), KSSYMLDLQPETT (SEQ. ID. NO: 5), KSSMHGDTPTLHEYMLDLQPETT (SEQ. ID. NO: 6), KSSLLMGTLGIVCPICSQKP (SEQ. ID. NO: 7), KVPRNQDWL (SEQ. ID. NO: 8), SYVDFFVWL (SEQ. ID. NO: 9), KYICNSSCM (SEQ. ID. NO: 10), and KSSKVPRNQDWL (SEQ. ID. NO: 11). In one embodiment, at least one antigen is RAHYNIVTF (SEQ. ID. NO: 1). In another embodiment, at least one antigen is GQAEPDRAHYNIVTF (SEQ. ID. NO: 2). In yet another embodiment, at least one antigen is KSSGQAEPDRAHYNIVTF (SEQ. ID. NO: 3). In some embodiments, KSSGQAEPDRAHYNIVTF (SEQ. ID. NO: 3) is modified to further comprise a hydrophobic group. In one embodiment, the hydrophobic group is a palmitoyl group.

In other embodiments, at least one antigen is YMLDLQPETT (SEQ. ID. NO: 4). In another embodiment, at least one antigen is KSSYMLDLQPETT (SEQ. ID. NO: 5). In yet another embodiment, KSSYMLDLQPETT (SEQ. ID. NO: 5) is modified to further comprise a hydrophobic group. In one embodiment, the hydrophobic group is a palmitoyl group.

In other embodiments, at least one antigen is KSSMHGDTPTLHEYMLDLQPETT (SEQ. ID. NO: 6). In another embodiment, KSSMHGDTPTLHEYMLDLQPETT (SEQ. ID. NO: 6) is modified to further comprise a hydrophobic group. In one embodiment, the hydrophobic group is a palmitoyl group.

In other embodiments, at least one antigen is KSSLLMGTLGIVCPICSQKP (SEQ. ID. NO: 7). In some embodiments, KSSLLMGTLGIVCPICSQKP (SEQ. ID. NO: 7) is modified to further comprise a hydrophobic group. In one embodiment, the hydrophobic group is a palmitoyl group.

In some embodiments, at least one antigen is KVPRNQDWL (SEQ. ID. NO: 8). In other embodiments, at least one antigen is SYVDFFVWL (SEQ. ID. NO: 9). In yet other embodiments, at least one antigen is KYICNSSCM (SEQ. ID. NO: 10). In another embodiment, at least one antigen is KSSKVPRNQDWL (SEQ. ID. NO: 11). In some embodiments, KSSKVPRNQDWL (SEQ. ID. NO: 11) is modified to further comprise a hydrophobic group. In one embodiment, the hydrophobic group is a palmitoyl group.

In various embodiments described herein, the vaccine formulation induces an immune response in a mammal by activating the mitogen-activated protein (MAP) kinase signaling pathway. Induction of an immune response by adjuvants such as cationic lipids are described, for example, in PCT/US2008/057678 (WO/2008/116078; "Stimulation of an Immune Response by Cationic Lipids") and PCT/US2009/040500 (WO/2009/129227; "Stimulation of an Immune Response by Enantiomers of Cationic Lipids"),. In some embodiments, the MAP kinase signaling pathway is activated by stimulating at least one of extracellular signal-regulated kinase ("ERK")-1, ERK-2, and p38. In other embodiments, the formulation enhances functional antigen-specific CD8+ T lymphocyte response. The term "mammal" is well known to those of skill in the art. In one embodiment, the mammal is a human.

In one embodiment described herein, a method of inducing an immune response in a mammal is provided. The method comprises the step of administering an effective amount of a vaccine formulation to the mammal, wherein the vaccine formulation comprises an adjuvant and an antigen assembly. The previously described embodiments of the vaccine formulation are applicable to the method of inducing an immune response in a mammal described herein.

In some embodiments, the immune response is activated via the MAP kinase signaling pathway in cells of the immune system of the mammal. In various embodiments, the MAP kinase signaling pathway is activated by stimulating at least one of ERK-1, ERK-2, and p38.

In other embodiments, the immune response activates cytotoxic T lymphocytes in the mammal. In one embodiment, the cytotoxic T lymphocytes are CD8+ T cells. In another embodiment, the administration enhances functional antigen-specific CD8+ T lymphocyte response. In yet another embodiment, the immune response activates an antibody response in the mammal. In other embodiments, the immune response activates interferon-gamma (IFN-γ) in the mammal.

In one embodiment described herein, a method of treating a disease in a mammal is provided. The method comprises the step of administering an effective amount of a vaccine formulation to the mammal, wherein the vaccine formulation comprises an adjuvant and an antigen assembly. The previously described embodiments of the vaccine formulation and of the method of inducing an immune response in a mammal are applicable to the method of treating a disease in an mammal described herein.

In some embodiments, "treatment," "treat," and "treating," as used herein with reference to infectious pathogens, refer to a prophylactic treatment which increases the resistance of a subject to infection with a pathogen or decreases the likelihood that the subject will become infected with the pathogen; and/or treatment after the subject has become infected in order to fight the infection, e.g., reduce or eliminate the infection or prevent it from becoming worse. In one embodiment, the method is a prophylactic treatment.

### EXAMPLE 1

### Preparation of Adjuvant and Adjuvants Incorporating an Antigen

Adjuvants may be prepared using cationic lipids alone. Alternatively, adjuvants may be prepared using mixtures of cationic lipids and other immunomodulators. Vaccine formulations may be prepared using a cationic lipid-based formulation incorporating an antigen. In the present example, DOTAP was used as an exemplary cationic lipid and HPV protein E7 peptide antigen was used as an exemplary antigen.

Sterile water for injection (WFI) or a buffer was used in all procedures in which cationic lipids were prepared into liposomes. In this example, liposomes were prepared using lipid films. The E7 antigen used for incorporation into the liposomes was an H-2D^{b} restricted CTL epitope (amino acid 49-57, RAHYNIVTF [SEQ. ID. NO. 1]) derived from HPV 16 E7 protein. Lipid films were made in glass vials by (1) dissolving the lipids in an organic solvent such as chloroform, and (2) evaporating the chloroform solution under a steady stream of dry nitrogen gas. Traces of organic solvent were removed by keeping the films under vacuum overnight. The lipid films were then hydrated by adding the required amount of WFI or buffer to make a final concentration of 4-10 mg/mL. The suspensions were then extruded to a size of 200 nm and stored at 4°C.

For the preparation of cationic lipid incorporating an antigen, the DOTAP lipid film was rehydrated by an aqueous solution of E7 peptide. Other methods used in general liposome preparation that are well known to those skilled in the art may also be used.

### EXAMPLE 2

### Preparation of Antigen Peptide Particulate Structures

Peptide sequences may be prepared as antigens for use with the present invention. In the present example, HPV protein E7 peptide antigen was used as an exemplary antigen. Peptide sequences may be selected for suitable hydrophilicity and may be modified by attaching a hydrophobic molecule or sequence to an N-terminal amino acid residue. For example, a hydrophobic chain such as a palmitic acid moiety may be covalently linked to the N-terminal amino acid residue of a peptide. The resulting antigen peptide particulate structures may be, for example, micelles or bilayers.

In this example, peptide sequences were selected and suspended in a suitable solvent at concentrations ranging from 20 to 50µg/µl. Other concentrations may be suitable based on the desired characteristics of a specific vaccine.

In this example, micelles or bilayers were made by diluting the stock solution of the lipidated peptide in a selected aqueous medium. These dilutions typically contain 0.5-2 mg/ml of a given peptide, but may vary depending on the amount of antigen required for the desired characteristics of a specific vaccine.

The peptide particulate structure may then be mixed 1:1 (v/v) with an empty liposome nanoparticle comprising cationic lipids.

### EXAMPLE 3

### Anti-tumor Efficacy of Cationic Lipid Adjuvants Compared with Traditional Adjuvants

The anti-tumor efficacy of cationic lipids used as adjuvants may be compared with traditional, well-known adjuvants known to induce antigen specific CTL activity. In this example, various lipid adjuvants were formulated as liposomes with HPV Protein E7 peptide antigen RAHYNIVTF (SEQ. ID. NO: 1) (aka "E7"). Various cationic lipids included DOTAP, DOTMA, and DOEPC. An anionic lipid included DOPG. Also in this example, the traditional, well-known adjuvants CpG and complete Freund adjuvant ("CFA") were also formulated with E7.

To compare the efficacy of cationic lipid/E7 formulations with other adjuvants to induce an immune response to a tumor, 6 to 12 tumor-bearing mice per formulation were treated six days after establishing tumors with E7 peptide formulated liposomes. The cationic lipid adjuvant formulations comprised cationic lipids (DOTAP, DOEPC and DOTMA) at 100 nmole dose composition of cationic lipid. The anionic lipid adjuvant formulation comprised DOPG. The traditional, well-known adjuvant formulations comprised well established strong adjuvants CFA or CpG ODN1826. Control groups included no treatment and e7 antigen only (i.e., no adjuvant).

Subcutaneous HPV-positive tumors were established in mice by injecting 10⁵ TC-1 cells into the flank of each mouse on day 0. On day 6, the mice received a single subcutaneous injection of the formulations in a 0.10 ml injection.

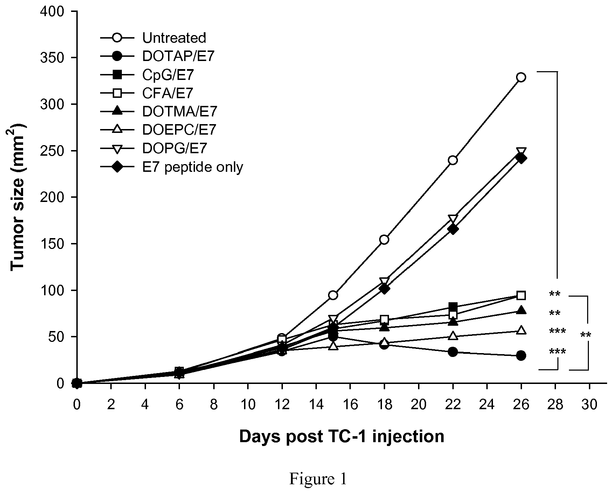

As shown in Figure 1, mice receiving the CFA or the CpG formulation and the various the cationic lipid formulations all demonstrated effective inhibition of tumor growth compared to the control groups on day 26. Mice that received the anionic lipid formulation did not show tumor regression. Mice receiving the cationic lipid-based formulations DOTAP/E7, DOTMA/E7 and DOEPC/E7 formulations exhibited better anti-cancer activity (p<0.01) compared to those formulated with the established adjuvant-based formulations CpG/E7 or CFA/E7.

### EXAMPLE 4

### Anti-Tumor Efficacy of Vaccine Formulations Comprising Cationic Lipid Nanoparticles and Antigen Assemblies

The anti-tumor efficacy of vaccine formulations can be evaluated by evaluating tumor regression. In this example, the vaccine formulation comprises cationic lipid nanoparticles and a peptide antigen assembly in a tubular structure. Furthermore, the exemplary cationic lipid in the present example is R-DOTAP and the exemplary antigen assembly is an HPV-16 E7 micelle.

In this example, H-2D^{b} restricted CTL epitope (amino acid 49-57, RAHYNIVTF [SEQ. ID. NO. 1]) derived from HPV 16 E7 protein was extended to amino acids 43-57, GQAEPDRAHYNIVTF, [SEQ. ID. No. 2]. SEQ. ID. No. 2 was then further extended with the amino acids KSS, and a hydrophobic palmitoyl chain was attached to the elongated peptide. As a result, micelle or bilayer formation was effectively promoted (i.e., palmitoyl-KSSGQAEPDRAHYNIVTF [SEQ. ID. No. 3]. SEQ. ID. No. 2 was observed to be a weak antigen when formulated and evaluated, similar to SEQ. ID. No. 1.

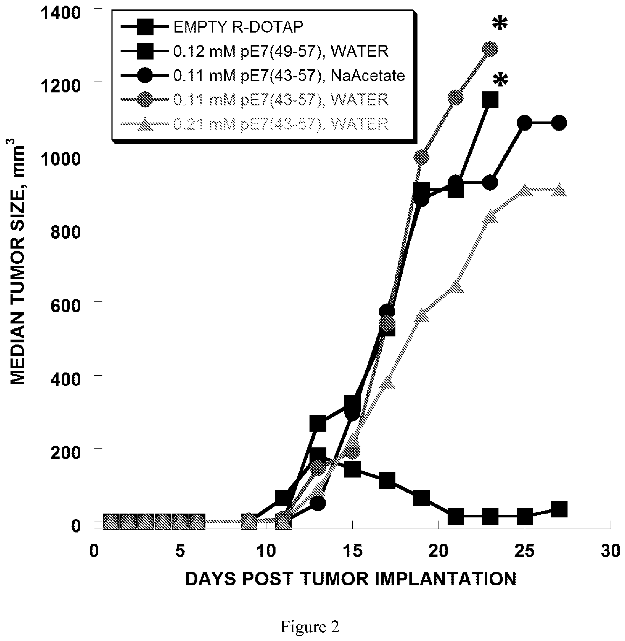

Approximately 0.2-0.4mg/ml (0.1-0.2mM) of the peptide antigen was encapsulated into 2mg/ml (2.9mM) of the liposome nanoparticles comprising R-DOTAP, resulting in a weak immune responses and a lack of effective tumor regression (see Figure 2). However, formulating the peptide antigen sequence into a particulate structure comprised only of SEQ. ID. No. 3 allows higher doses of the antigen to be delivered compared to delivery via a cationic lipid adjuvant delivery system. Thus, an effective means of overcoming the weak antigenicity of the peptide can be obtained.

To evaluate this approach, HPV-positive tumors were established as described in Example 3 above. On day 6, the mice (5 per group) received a single subcutaneous injection of various vaccine formulations:
- Formulation 1 (Negative control): Empty liposome nanoparticles comprising R-DOTAP.
- Formulation 2: A mixture of 2.3mg/ml of R-DOTAP liposomal nanoparticles and 1.1mg/ml of SEQ. ID. No. 3 peptide antigen assembly as micelles (0.10 ml injection).
- Formulation 3: A mixture of 2.3mg/ml of S-DOTAP liposomal nanoparticles and 1.1mg/ml of SEQ. ID. No. 3 peptide antigen assembly as micelles (0.10 ml injection).

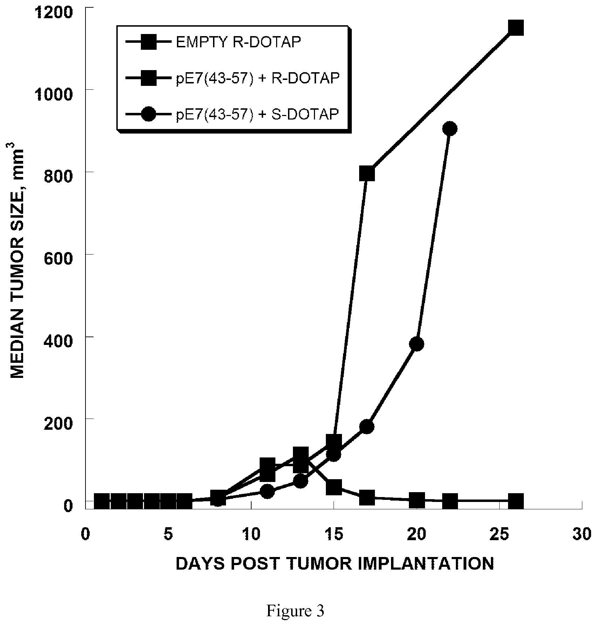

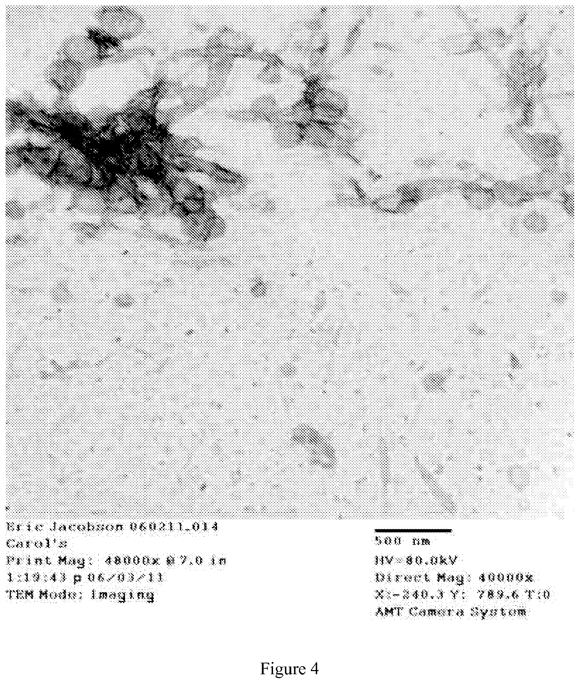

Figure 3 shows an effective tumor regression in mice injected with Formulation 2. Negative stain scanning electron microscopy shows the presence of a mixture of an antigen assembly in tubular micelle structures and an adjuvant in spherical R-DOTAP liposome nanoparticles co-existing in the formulated vaccine mixture (Figure 4). The present example demonstrates that a vaccine formulation comprising an adjuvant (e.g., R-DOTAP liposomal nanoparticles) and an antigen assembly (e.g., HPV E7 peptide antigen as micelle particles) can effectively promote tumor regression in an animal.

### EXAMPLE 5

### Immune Response in Humanized HLA-A2 Transgenic Mice Using Vaccine Formulations Comprising Cationic Lipid Nanoparticles and Antigen Assemblies Containing Single Peptide Antigens

Induction of interferon-y (IFN-γ) is known to result from activated antigen-specific cytotoxic T-lymphocytes (CD8+ T-cells) and is important for development of an effective therapeutic immune response in an animal. Immune responses in humanized HLA-A2 transgenic mice using vaccine formulations comprising cationic lipid nanoparticles and antigen assemblies can be evaluated by measuring induction of IFN-γ by an enzyme-linked immunosorbent spot (ELISPOT) assay. In this example, the vaccine formulation comprises cationic lipid nanoparticles (e.g., R-DOTAP) and a peptide antigen assembly of various compositions and structures.

Two different vaccine formulations were evaluated in the present example. Formulation 1 comprised the cationic lipid R-DOTAP adjuvant nanoparticles and utilized the well established HPV-16 E7 HLA-A2 antigenic human peptide antigen YMLDLQPETT [SEQ. ID. No. 4]. SEQ. ID. No. 4 was modified by attaching 3 amino acids and palmitic acid to obtain the sequence palmitoyl-KSSYMLDLQPETT [SEQ. ID. No. 5]. Particulate peptide structures were spontaneously formed according to the methods described herein. Formulation 1 contained an adjuvant of approximately 2.8mg/ml of R-DOTAP adjuvant nanoparticles and an antigen assembly of approximately 0.83mg/ml of SEQ. ID. No. 5 peptide.

Mice were injected with 0.1ml of Formulation 1 on days 0 and 7. The mice were sacrificed and splenocytes removed from each mouse for evaluation on day 14. The splenocytes were harvested from the immunized mice and seeded into wells of a 96-well plate (approximately 250,000 splenocytes per well). The individual wells were then exposed to peptide antigen YMLDLQPETT [SEQ. ID. No. 4] and the immune response was analyzed. Each spot that developed in the assay represents a single reactive splenocyte cell, and the readout of the analysis provides the number of spots formed on the 96-well plate. Thus, the ELISPOT assay provided a quantitative assay to effectively determine the resulting immune response to the antigen YMLDLQPETT. The results of the ELISPOT assay, demonstrating a high efficacy of Formulation 1, are shown in Table 1.

**Table 1: Immune Response as Measured by ELISPOT**

| Formulation Number | Peptide Antigen Sequence/Composition | Resulting Antigen Particle Structure | Average # of IFN-γ Spots per 250,000 splenocytes |
|---|---|---|---|
| #1 | - Palmitoyl- KSSYMLDLQPETT | Spherical | 262.2 |
| #2 | - Palmitoyl-KSSMHGDTPTLHEYMLDLQPETT | Tubular | 122.4 |

Formulation 2 comprised the cationic lipid R-DOTAP adjuvant nanoparticles and utilized a peptide that selected the first 20 amino acids from the N-terminus of the HPV-16 E7 protein. This peptide was modified to palmitoyl-KSSMHGDTPTLHEYMLDLQPETT [SEQ. ID. No. 6]. Particulate peptide structures were spontaneously formed according to the methods described herein. Formulation 2 contained an adjuvant of approximately 2.8mg/ml of R-DOTAP adjuvant nanoparticles and an antigen assembly of approximately 0.95mg/ml of SEQ. ID. No. 6 peptide.

Mice were injected with 0.1ml of Formulation 2 on days 0 and 7. The mice were sacrificed and splenocytes removed from each mouse for evaluation on day 14. Again, as shown in Table 1, a strong immune response was demonstrated in response to immunization with Formulation 2.

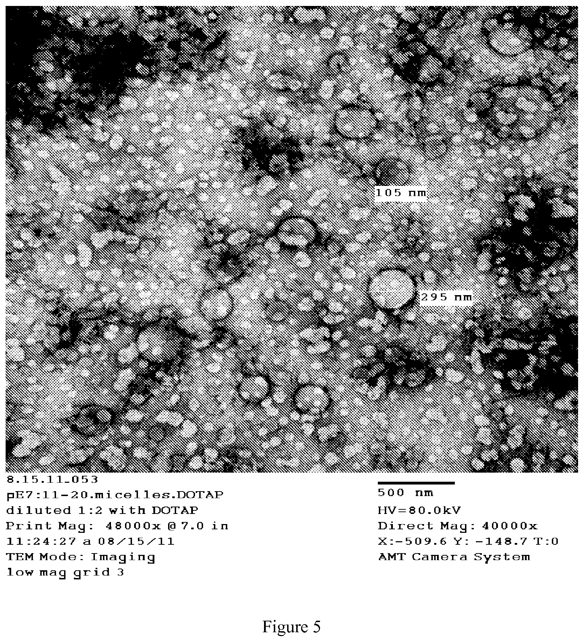

Negative stain scanning electron microscopy showed the presence of a mixture of an antigen assembly in spherical micelle structures comprising palmitoyl-KSSYMLDLQPETT and an adjuvant comprising spherical R-DOTAP liposome nanoparticles (i.e. Formulation 1), co-existing in the formulated mixture (see Figure 5).

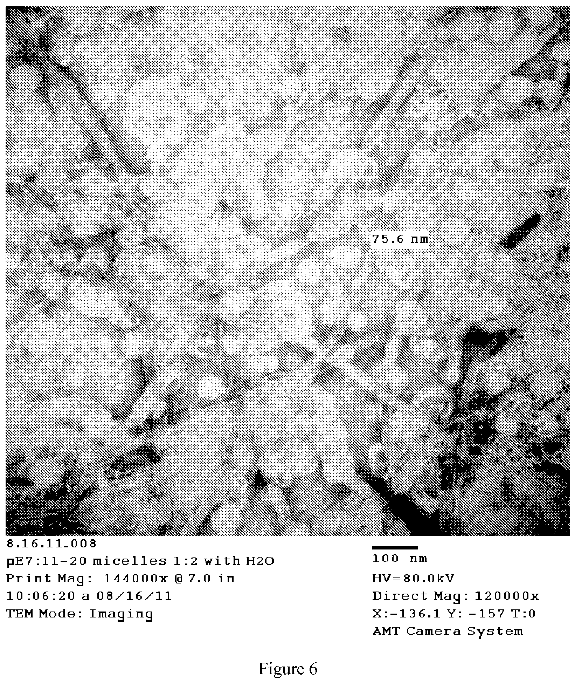

Figure 6 shows an antigen assembly in tubular micelle structures comprising palmitoyl-KSSMHGDTPTLHEYMLDLQPETT and an adjuvant comprising spherical R-DOTAP liposome nanoparticles (i.e. Formulation 2), co-existing in the formulated mixture.

### EXAMPLE 6

### Immune Response in Humanized HLA-A2 Transgenic Mice Using Vaccine Formulations Comprising Cationic Lipid Nanoparticles and Antigen Assemblies Containing Multiple Peptide Antigens

Similar to Example 5, ELIPSOT can be utilized to evaluate the efficacy of a vaccine formulation comprising cationic lipid nanoparticles and antigen assemblies. In this example, the vaccine formulation comprises cationic lipid nanoparticles (e.g., R-DOTAP) and a peptide antigen assembly comprising three peptide antigens.

The formulation in the present example comprised the cationic lipid R-DOTAP adjuvant nanoparticles and an antigen assembly of a mixed micellar structure comprising three peptide antigens. An immune response to the peptide sequence YMLDLQPETT was evaluated. The mixed peptide particles were composed of SEQ. ID. No. 5, SEQ. ID. No. 6, and palmitoyl-KSSLLMGTLGIVCPICSQKP [SEQ. ID. No. 7]. This formulation contained an adjuvant of approximately 2.8mg/ml of R-DOTAP adjuvant nanoparticles and an antigen assembly of approximately 1mg/mL of each peptide.

Mice were injected with 0.1ml of the formulation on days 0 and 7. The mice were sacrificed and splenocytes removed from each mouse for evaluation on day 14. As shown in Table 2, a strong immune response was demonstrated in response to immunization with this formulation.

**Table 2: Immune Response as Measured by ELISPOT**

| Peptide Antigen Sequence/Composition | Resulting Antigen Particle Structure | Average # of IFN-γ Spots per 250,000 splenocytes |
|---|---|---|
| - Palmitoyl-KSSGQAEPDRAHYNIVTF, - Palmitoyl-KSSMHGDTPTLHEYMLDLQPETT, and - Palmitoyl-KSSLLMGTLGIVCPICSQKP | Tubular | 447.1 |

### EXAMPLE 7

### Immune Response in Using Vaccine Formulations Comprising Cationic Lipid Nanoparticles and Antigen Assemblies Containing Micellar Melanoma Antigens

Immune responses in C57/BL6 mice using vaccine formulations comprising cationic lipid nanoparticles and antigen assemblies can be evaluated by ELISPOT. In this example, the vaccine formulation comprises cationic lipid nanoparticles (e.g., R-DOTAP) and a peptide antigen assembly of melanoma antigens in a micellar structure.

In this example, Formulation A comprised the cationic lipid R-DOTAP adjuvant nanoparticles and an antigen assembly encapsulating three melanoma antigens: gp100 (KVPRNQDWL [SEQ. ID. No. 8]), TRP2 (SYVDFFVWL [SEQ. ID. No. 9]), and p53 (KYICNSSCM [SEQ. ID. No. 10]). Formulation A contained an adjuvant of approximately 4.3mM of R-DOTAP adjuvant nanoparticles and approximately 0.25mM of gp100 peptide.

In order to deliver larger amounts of gp100 antigen, a micellar formulation was developed. The modified antigen palmitoyl-KSS KVPRNQDWL [SEQ. ID. No. 11] was used to develop a micellar formulation. Formulation B comprised about 4.4mM of liposomal R-DOTAP encapsulated TRP2 and a micellar formulation of approximately 0.46mM of gp100. Micelles made from the modified antigen were prepared as described in Example 2. The effectiveness of the vaccines were evaluated in C57/BL6 mice by ELISPOT as described in Example 5.

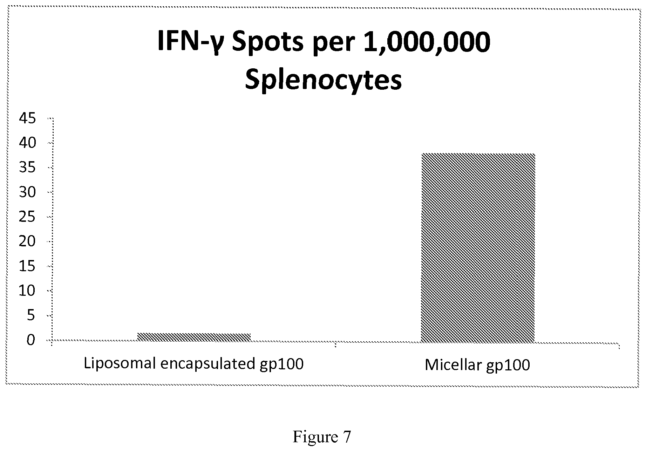

Figure 7 shows a greater than 20-fold increase in immune response to the formulation comprising gp100 antigen in a micellar formulation compared to gp100 antigen that was liposomally encapsulated. The immune response is believed to be enhanced due to the approximate doubling of the amount of gp100antigen delivered via the micellar formulation.

### EXAMPLE 8

### Comparison of Immune Response In Vaccine Formulations Using Micellar Antigen Assemblies and Liposomally Encapsulated Antigen Assemblies

Immune response using vaccine formulations comprising varying cationic lipid nanoparticles and varying antigen assemblies can be evaluated by ELISPOT. In this example, the vaccine formulations can be formulated using various cationic lipid nanoparticles (e.g., DOEPC or DOTMA) or the emulsion adjuvant Montanide. Furthermore, the vaccine formulations can be formulated using an antigen assembly in either a micellar structure or a liposomally encapsulated structure.

Various different vaccine formulations were evaluated in the present example. In one formulation, the antigen assembly comprised the peptide antigen [SEQ. ID. No. 2] (0.11mM) and the cationic lipid adjuvant DOEPC (ImM). In a second formulation, the antigen assembly comprised the modified peptide antigen [SEQ. ID. No. 3] (0.11mM) to enable micelle formation, and the cationic lipid adjuvant DOEPC (1mM). In a third formulation, the antigen assembly comprised the peptide antigen [SEQ. ID. No. 2] (0.11mM) and the cationic lipid adjuvant DOTMA (ImM). In a fourth formulation, the antigen assembly comprised the modified peptide antigen [SEQ. ID. No. 3] (0.11mM) and the cationic lipid adjuvant DOTMA (1mM). In a fifth formulation, the antigen assembly comprised the peptide antigen [SEQ. ID. No. 2] (0.11mM) and the emulsion adjuvant Montanide. In a sixth formulation, the antigen assembly comprised the modified peptide antigen [SEQ. ID. No. 3] (0.11mM) and the emulsion adjuvant Montanide.

In formulations where the antigen assembly comprised the unmodified peptide antigen [SEQ. ID. No. 2], the antigen assembly was formulated as liposomally encapsulated. In comparison, in formulations where the antigen assembly comprised the modified peptide antigen [SEQ. ID. No. 3], the antigen assembly was formulated as micellar and was mixed with the adjuvant at a 1:1 ratio in order to maintain identical antigen and adjuvant content compared to the corresponding liposomal formulations. The liposomally encapsulated antigen assemblies and the micellar antigen assemblies were made according to the protocols of Example 1 and Example 2, respectively. Potency of the various vaccine formulations was evaluated by determining the antigen-specific immune response via ELISPOT.

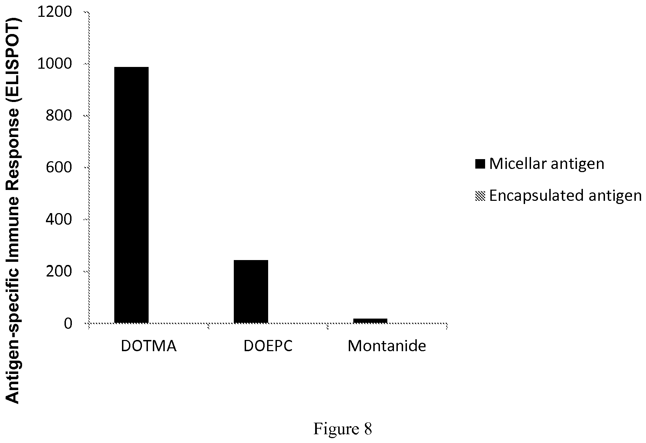

Figure 8 shows the results of the present example. Identical doses of antigen and adjuvant lead to vastly superior antigen-specific immune responses when the antigen assembly is delivered in micellar form with a specific adjuvant. Importantly, the observed immune response is very weak when using antigen assembly delivered in the liposomally encapsulated form.

The observed immune response is also dependent on the specific adjuvant administered with the micellar antigen as seen with DOTMA, DOEPC and Montanide. The DOTMA formulations demonstrated superior immune response to the DOEPC formulations, and both cationic lipid formulations were superior to the emulsion adjuvant Montanide.

## Claims

1. A vaccine formulation comprising a cationic lipid adjuvant particle and a self-forming particulate protein or peptide antigen assembly, wherein the antigen assembly comprises a micellar structure and wherein the micellar structure is formed by attaching a hydrophobic molecule or sequence to an N-terminal amino acid residue of a protein or peptide antigen via a linker.

2. The vaccine formulation of claim 1, wherein the cationic lipid is an immunomodulator.

3. The vaccine formulation of claim 1 or claim 2, wherein the cationic lipid is selected from the group consisting of DOTAP, DOTMA, DOEPC, and combinations thereof.

4. The vaccine formulation of any one of claims 1 to 3, wherein the cationic lipid is DOTAP.

5. The vaccine formulation of any one of claims 1 to 4, wherein the cationic lipid is an enantiomer of the cationic lipid.

6. The vaccine formulation of claim 5, wherein the enantiomer is R-DOTAP.

7. The vaccine formulation of any one of claims 1 to 6, wherein the self-forming antigen assembly comprises one or more protein or peptide antigens selected from the group consisting of a cancer antigen, a viral antigen, a bacterial antigen, and a pathogenic antigen.

8. The vaccine formulation of any one of claims 1 to 7, wherein at least one antigen is an HPV protein or peptide.

9. The vaccine formulation of any one of claims 1 to 8, wherein at least one antigen is a melanoma antigen.

10. The vaccine formulation of any one of claims 1 to 9, wherein one or more antigens is an antigen modified to increase or decrease hydrophobicity of the antigen to cause micelle formation.

11. The vaccine formulation of any of claims 1 to 10, for use in inducing an immune response in a mammal.

## Patentansprüche

1. Eine Impfstoffformulierung mit einem Kationisches-Lipid-Hilfsmittelteilchen und einer sich selbst bildenden partikulären Protein- oder Peptidantigen-Anordnung, wobei die Antigen-Anordnung eine Mizellar-Struktur aufweist, und wobei die Mizellar-Struktur durch Anlagern eines hydrophoben Moleküls oder einer derartigen Sequenz an einem N-endständigen Aminosäurerest eines Protein- oder Peptidantigens über ein Bindeelement gebildet wird.

2. Die Impfstoffformulierung gemäß Anspruch 1, bei der das kationische Lipid ein Immunmodulator ist.

3. Die Impfstoffformulierung gemäß Anspruch 1 oder Anspruch 2, bei der das kationische Lipid aus der Gruppe ausgewählt ist, die besteht aus DOTAP, DOTMA, DOEPC und Kombinationen daraus.

4. Die Impfstoffformulierung gemäß einem der Ansprüche 1 bis 3, bei der das kationische Lipid DOTAP ist.

5. Die Impfstoffformulierung gemäß einem der Ansprüche 1 bis 4, bei der das kationische Lipid ein Enantiomer des kationischen Lipids ist.

6. Die Impfstoffformulierung gemäß Anspruch 5, bei der das Enantiomer R-DOTAP ist.

7. Die Impfstoffformulierung gemäß einem der Ansprüche 1 bis 6, bei der die sich selbst bildende Antigen-Anordnung ein oder mehrere Protein- oder Peptidantigene aufweist, die ausgewählt sind aus der Gruppe, die besteht aus einem Krebs-Antigen, einem viralen Antigen, einem bakteriellen Antigen und einem pathogenen Antigen.

8. Die Impfstoffformulierung gemäß einem der Ansprüche 1 bis 7, bei der zumindest ein Antigen ein HPV-Protein oder-Peptid ist.

9. Die Impfstoffformulierung gemäß einem der Ansprüche 1 bis 8, bei der zumindest ein Antigen ein Melanom-Antigen ist.

10. Die Impfstoffformulierung gemäß einem der Ansprüche 1 bis 9, bei der ein oder mehrere Antigene ein Antigen ist/sind, das modifiziert ist, um eine Hydrophobie des Antigens zu erhöhen oder zu senken, um eine Mizellenbildung hervorzurufen.

11. Die Impfstoffformulierung gemäß einem der Ansprüche 1 bis 10 zur Verwendung beim Induzieren einer Immunantwort bei einem Säugetier.

## Revendications

1. Formulation de vaccin comprenant une particule d'adjuvant de lipide cationique et un assemblage d'antigène protéique ou peptidique particulaire auto-formé, où l'assemblage d'antigène comprend une structure micellaire et où la structure micellaire est formée en attachant une molécule ou une séquence hydrophobe à un résidu d'acide aminé N-terminal d'un antigène protéique ou peptidique via un segment de liaison.

2. Formulation de vaccin selon la revendication 1, dans laquelle le lipide cationique est un immunomodulateur.

3. Formulation de vaccin selon la revendication 1 ou la revendication 2, dans laquelle le lipide cationique est sélectionné dans le groupe consistant en le DOTAP, le DOTMA, la DOEPC, et des combinaisons de ceux-ci.

4. Formulation de vaccin selon l'une quelconque des revendications 1 à 3, dans laquelle le lipide cationique est le DOTAP.

5. Formulation de vaccin selon l'une quelconque des revendications 1 à 4, dans laquelle le lipide cationique est un énantiomère du lipide cationique.

6. Formulation de vaccin selon la revendication 5, dans laquelle l'énantiomère est le R-DOTAP.

7. Formulation de vaccin selon l'une quelconque des revendications 1 à 6, dans laquelle l'assemblage d'antigène auto-formé comprend un ou plusieurs antigènes protéiques ou peptidiques sélectionnés dans le groupe consistant en un antigène cancéreux, un antigène viral, un antigène bactérien, et un antigène pathogène.

8. Formulation de vaccin selon l'une quelconque des revendications 1 à 7, dans laquelle au moins un antigène est une protéine ou un peptide du HPV.

9. Formulation de vaccin selon l'une quelconque des revendications 1 à 8, dans laquelle au moins un antigène est un antigène de mélanome.

10. Formulation de vaccin selon l'une quelconque des revendications 1 à 9, dans laquelle un ou plusieurs antigènes est un antigène modifié pour augmenter ou réduire l'hydrophobicité de l'antigène afin de provoquer la formation de micelles.

11. Formulation de vaccin selon l'une quelconque des revendications 1 à 10, destinée à être utilisée pour induire une réponse immunitaire chez un mammifère.
